# EUROPÄISCHE PATENTSCHRIFT

(11) **EP 1 077 050 B1**
(45) Veröffentlichungstag und Bekanntmachung des Hinweises auf die Patenterteilung: **26.11.2003**
(21) Anmeldenummer: 00116575.2
(22) Anmeldetag: 01.08.2000
(51) Int. Cl.: A61F 5/058

(54) **Clavicula-Bandage**
Clavicle bandage
Bandage claviculaire

(30) Priorität: 17.08.1999 DE 19939005
(43) Veröffentlichungstag der Anmeldung: 21.02.2001
(73) Patentinhaber: Beiersdorf AG, 20253 Hamburg (DE)
(72) Erfinder: Herzberg, Thorsten, 22419 Hamburg (DE); Shanahan, John, Thurles, Co Tipperary (IE)

(56) Entgegenhaltungen:
- EP-A- 0 379 929
- US-A- 3 382 868
- US-A- 3 856 004
- US-A- 3 897 776
- US-A- 4 589 406

## Beschreibung

Die Erfindung betrifft eine medizinische Bandage zur Behandlung von Verletzungen, insbesondere Frakturen des Schlüsselbeins (Clavicula).

Frakturen des Schlüsselbeins und andere Verletzungen desselben werden zum überwiegenden Teil durch Bandagen oder Verbände behandelt. Bekannt ist der "Rucksack"-Verband, bei dem beispielsweise ein Trikotschlauch mit Watte verfüllt und auf passende Länge zugeschnitten wird. Dieser gefüllte Schlauch wird am Nackenbereich des Patienten angelegt, über beide Schlüsselbeine und Achselhöhlen geführt und auf dem Rücken verknotet.
Nachteilig bei dieser Art der Bandage ist, daß die Herstellung in der Klinik selbst erfolgen muß, was sich als sehr zeitintensiv herausstellt. Des weiteren ist der Verband dehnfähig, d.h., unter Zug längt er sich, was dazu führt, daß der Verband mehrmals im Laufe der Behandlung nachgestellt werden muß, um die Bruchstücke des Schlüsselbeins fest zu positionieren. Schließlich besteht die Gefahr, daß sich aufgrund des Knotens im Rückenbereich auf der Haut Reizungen oder gar Verletzungen ergeben. Erschwerend kommt hinzu, daß der Verband nicht besonders schweißabsorbierend ist. Übermäßiges Schwitzen unter dem Verband kann daher zu weiteren Hautirritationen führen.

Die US 3,856,004 offenbart eine Clavicula-Bandage, die im wesentlichen aus einem Streifen gebildet ist. Der Streifen ist mittig bis auf ein kleines Endstück in zwei Zügel geteilt. Am Ende des Einschnitts zwischen den Zügeln befindet sich ein gleichseitiges Dreieck, das sich auf dem Rücken des Patienten befindet und durch das beim Anlegen der Bandage die Zügel gezogen und auf sich selbst mittels Klettverschlüssen fixiert werden.

Die US 3,897,776 beschreibt eine Clavicula-Bandage, die der aus der US 3,856,004 bekannten ähnlich ist. Die Bandage wird von zwei Zügeln gebildet, die auf dem Rücken mit Hilfe eines annähernd rechtwinkligen Dreiecks fixiert werden.

Aus der DE 259 349 ist eine als "Geradehalter" bezeichnete Vorrichtung bekannt, die aus zwei auf dem Rücken zusammenlaufenden Armschlaufen besteht, wobei die oberhalb der Schultern nach hinten laufenden Enden von einem festen Ring gehalten sind. Vom Ring hängt ein elastischer Gurt herab, durch dessen am unteren Ende vorhandene Öse die beiden anderen unterhalb der Schultern nach hinten laufenden, mit verstellbaren Ösen ausgestatteten Enden kreuzweise hindurchgeführt sind.

Die geschilderten Bandagen oder Verbände können die an sie gestellten Anforderungen allerdings nur eingeschränkt erfüllen. Zum Teil sind sie sehr aufwendig im Herstellungsprozeß, können nur unzureichend nachgespannt werden und weisen Verschlüsse auf, die teuer und unkomfortabel zu handhaben sind.

Die DE 298 08 357 legt eine Clavicula-Bandage dar, deren Hauptbestandteil zwei Bänder sind, die von einem Befestigungspunkt eines Rückenteils ausgehen, über jeweils eine Schulter des Patienten auf die Körpervorderseite und von dort unter den Achseln hindurch zu diesem Befestigungspunkt am Rückteil geführt werden. Die Bänder sind im Brustbereich durch ein Zugelement miteinander verbunden, wobei die Länge des Zugelements so bemessen und einstellbar ist, daß die Bänder zur Körpermitte hin gelenkt werden.

In der EP 0 379 929 B1 sind Clavicula-Bandagen gezeigt, die aus zwei in ihrer Länge einstellbaren Gurten gebildet sind. Die Gurte weisen an ihren freien Enden Mittel zur Schlaufenbildung auf. Die Gurte sind mit ihrem anderen Ende an einem Ring, der vorzugsweise mit einem flachen Querschnitt ausgeführt ist, unabhängig voneinander befestigt. Weiterhin bestehen die Gurte aus im wesentlichen nicht dehnfähigem Material. Bei angelegter Bandage können im Bereich der Achselhöhlen Polster an der Bandage vorhanden sein. Beim Anlegen der Bandage drücken die Gurte auf die Clavicula und sind auf dem Rücken miteinander verbunden.

Die Konstruktion, wie sie in EP 0379 929 B1 erwähnt wird, hat sich über einen langen Zeitraum bewährt. Es hat sich allerdings herausgestellt, daß die Gurte vom Rücken kommend meistens schlecht oder ungenau positioniert sind und im angelegten Zustand zunehmend nach lateral wandern, was die Funktion und den Tragekomfort wesentlich verschlechtert. Der Ring führt im Bereich der medialen Schulterblattgräten zu Druckstellen und durch teilweise Verformung zu einem Verlust der Stabilität der Bandage. Des weiteren sind die Art und Ausführung der Achselpolster verbesserungswürdig, da im achsilaren Bereich oftmals Druckstellen entstehen und die Arme in eine Abduktionsstellung gezwungen werden, was oftmals in Schmerzen, hervorgerufen durch Druck auf die Achsilar-Nerven, mündet.

Aufgabe der Erfindung ist es, eine Clavicula-Bandage zu entwickeln, die abgeleitet vom traditionellen Rucksackverband aus Trikotschlauch die oben erwähnten Mängel nicht aufweist. Insbesondere soll die Bandage problemlos anzulegen und einen hohen Tragekomfort bieten, dann einfach nachzujustieren sein, falls dies erforderlich ist.

Gelöst wird diese Aufgabe durch eine Clavicula-Bandage, wie sie im Hauptanspruch niedergelegt ist. Gegenstand der Unteransprüche sind dabei vorteilhafte Weiterbildungen der Clavicula-Bandage.

Demgemäß betrifft die Erfindung eine Clavicula-Bandage mit zwei in ihrer Länge einstellbaren Bändern, die an ihren freien Enden Mittel zur Schlaufenbildung aufweisen, wobei die Bänder, die im wesentlichen aus nicht dehnbarem Material bestehen, jeweils mit ihrem anderen Ende an einem Ring befestigt sind und die Bänder durch eine Stofftasche in ihrer Lage zueinander fixiert sind.

Vorzugsweise bestehen die Bänder aus einem laminierten Schaumstoff. Es hat sich als vorteilhaft erwiesen, wenn die Bänder einen hohen Polstereffekt unter Belastung von ungefähr 50 N eine bevorzugte Längsdehnung von kleiner als 35%, besonders bevorzugt eine Längsdehnung von kleiner als 10 %, aufweisen.

Der Ring weist einen flachen elliptischen Querschnitt beziehungsweise ein elliptisches Profil auf. Dadurch liegt der Ring bei angelegter Bandage, eben bedingt durch das elliptische Profil, flächig auf, so daß ein Kanten- oder Randdruck des Ringes auf den Rücken des Patienten vermieden wird. Die Profilierung des Ringes erhöht außerdem die Stabilität, und eine Verformung des Ringes im angelegten Zustand ist nicht zu beobachten.
Der Ring sollte aus üblichen Kunststoffen wie Polypropylen oder Polyethylen bestehen, um allergische Reaktionen auf der Haut zu minimieren.

In einer ersten bevorzugten Ausführungsform bestehen die Mittel zur Schlaufenbildung aus einem Klettverschlußsystem.
Weiter vorzugsweise ist das Klettverschlußsystem in der Weise ausgebildet, daß sich am freien Ende der Bänder auf ihrer körperabgewandten Außenfläche ein Klettenabschnitt befindet, der das eine Verschlußteil des Klettverschlusses bildet und aus im Abstand voneinander angeordneten aufwärts gerichteten Häkchen besteht, und daß sich im Anschluß daran zumindest auf einem Teilabschnitt der Außenfläche klettbares Material befindet, welches als das andere Teil des Klettverschlusses fungiert, indem die Häkchen auf ihm zu haften vermögen.
Der klettbare Teilabschnitt kann mindestens ein Sechstel der Länge der Bänder ausmachen. In einer alternativen Ausführungsform bestehen die Bänder auf ihrer körperabgewandten Außenfläche über die ganze Länge aus einem Material, auf welchem die Häkchen des anderen Verschlußteils haften.

Die Stofftasche, welche aus einem um den Ring umgeschlagenen Stoffabschnitt gebildet wird, weist zur Fixierung der Bänder am Ring vorteilhafterweise die Form eines nicht regelmäßigen Pentagons auf, so daß der Winkel a zwischen den sich vom Ring entfernenden Bändern 50° bis 95° beträgt, insbesondere 68°.
Die Fixierung erfolgt üblicherweise durch das Vernähen der Stofftasche mit den Bändern. Durch die Ausbildung einer solchen Tasche wird bei angelegter Bandage eine verbesserte Gurtführung im Vergleich zu den bekannten Bandagen erzielt, wobei die kraniale Spitze des Pentagons höher als bei bekannten Bandagen liegt beziehungsweise näher am Nackenbereich des Patienten. Der Ring endet in seiner optimalen Position im Bereich unterhalb des siebten Halswirbels, insbesondere zwischen den ersten vier Brustwirbeln (Vertebrae thoracles Th1 bis Th4).
Die Gurtbefestigung mittels der Stofftasche wirkt einer Lateralisierung der Bänder im frontalen Bereich entgegen. Zusätzlich ergibt sich durch diese Art der Befestigung eine Polsterung des elliptischen Ringes.

In einer weiteren vorteilhaften Ausführungsform der erfindungsgemäßen Clavicula-Bandage weisen die Bänder etwa im Mittelabschnitt ihrer körperzugewandten Innenseite eine Polsterung aus einem schweißaufsaugenden Material wie Polyamid, Polyester und/oder Baumwolle auf, vorzugsweise aus Baumwolle.

Die Polsterung im Bereich der Achseln besteht insbesondere aus einem Abstandsgewirke mit einer Stärke von 3,5 mm bis 15 mm, vorzugsweise 5 mm, und einem Flächengewicht von 200 bis 1000 g/m², vorzugsweise 300 bis 800 g/m², wobei weiter bevorzugt die Oberfläche der der Achselpartie zugewandten Seite der Polsterung ähnlich wie ein Fleece aufgerauht ist, so daß ein optimaler Polstereffekt resultiert. Die Aufrauhung der Oberfläche erzeugt eine besonders weiche Oberfläche.
Die hohe Luftdurchlässigkeit des Abstandsgewirkes, insbesondere mit der aufgerauhten Oberfläche, sorgt für eine gute Atmung der Haut, und der Schweiß wird von der Oberfläche zum Inneren des Bandagenbandes transportiert.
Trotz des sehr hohen Polstereffekts wird eine Gefahr des Hitzestaus vermieden.
Die Achselpolster können zur weiteren Steigerung an der Oberfläche zur Haut auch spezielle Fasern enthalten, die ähnlich wie ein Docht den Schweiß von der Körperseite zum Gurt hin transportieren, wie zum Beispiel Cool max.

In einer weiteren Ausführungsform können die Achselpolster aus einem Abstandsgewebe bestehen, das über eine zusätzliche Abdeckung zu der der Achselpartie zugewandten Seite verfügt.

Die Bandage kann in unterschiedlichen Größen dargeboten werden. Die Gesamtlänge schwankt zwischen etwa 1 m bis 2,1 m, wobei die Klettabschnitte an den Bändern ungefähr 10 bis 15 cm einnehmen. Die Polsterabschnitte erstrecken sich über 15 bis 45 cm, insbesondere über 25 bis 35 cm.

Die Bänder der Clavicula-Bandage überschreiten vorzugsweise nicht eine Dicke von 2 cm.
Der Ring hat einen Außendurchmesser von 8 cm und einen Innendurchmesser von 6 cm, wobei der Ring mittig eine Höhe von 0,35 cm bis 0,60 cm, bevorzugt 0,40 cm bis 0,50 cm aufweist.

Überraschend und unerwartet zeigt die Bandage in der Therapie einen sehr hohen Gewinn gegenüber den bisher bekannten. Eine Reizung der Haut auf dem Rücken unterhalb des Ringes ist nicht zu beobachten, obwohl der Ring mit Druck aufliegt. Die bevorzugte Wahl des angegebenen Materials für die Bänder und die Polster bewirkt, daß die Bandage einen hohen Tragekomfort bietet und daß dieselbe nur wenig nachgespannt werden muß. Des weiteren bedingen die ausgewählten Materialien ein ansprechendes Design.

Anhand der nachfolgend beschriebenen Figuren wird eine besonders vorteilhafte Ausführung der Clavicula-Bandage näher erläutert, ohne damit die Erfindung unnötig einschränken zu wollen. Es zeigen
- Figur 1: die Clavicula-Bandage in einer besonders vorteilhaft ausgeführten Ausführungsform,
- Figur 2: Details zur Stofftasche, die die Bänder am Ring festlegt, sowie einen seitlichen Schnitt gemäß der Linie A-A durch diesen Bereich der Bandage,
- Figur 3: die am menschlichen Körper angelegte Clavicula-Bandage von anterior und
- Figur 4: die am menschlichen Körper angelegte Clavicula-Bandage von posterior.

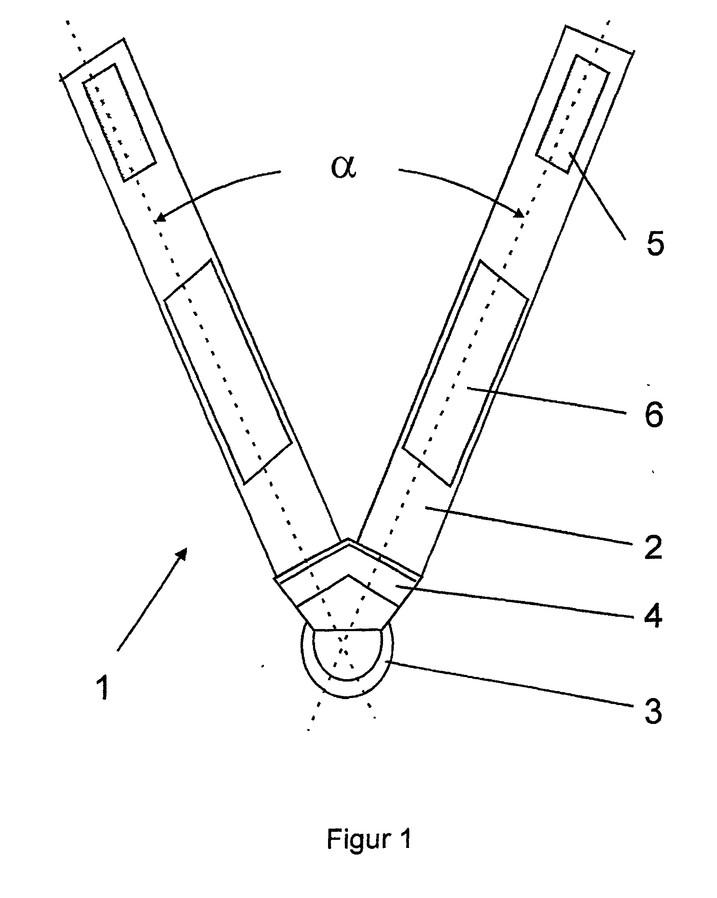

Die Figur 1 zeigt die erfindungsgemäße Clavicula-Bandage 1 mit den zwei in ihrer Länge einstellbaren Bändern 2, die an ihren freien Enden Mittel 5 zur Schlaufenbildung aufweisen. Die Mittel 5 sind als Klettverschlußsystem in der Weise ausgebildet, daß sich am freien Ende der Bänder 2 auf ihrer körperabgewandten Außenfläche jeweils ein Klettenabschnitt 5 befindet, die das eine Verschlußteil des Klettverschlusses bilden und die beim Anlegen der Bandage 1 auf dieser befestigt werden.

Mit dem anderen Ende sind die Bänder 2 an einem Ring 3 befestigt, der einen flachen elliptischen Querschnitt aufweist Die Fixierung der Bänder 2 am Ring 3 erfolgt durch eine Stofftasche 4, die in Form eines leicht verzerrten Pentagons ausgeführt ist.

Zwischen den Bändern 2 ergibt sich somit ein Winkel α von circa 68°.

Weiterhin sind an den Bändern 2, und zwar ungefähr im Mittelabschnitt ihrer körperzugewandten Innenseite, Polsterungen 6 aus einem schweißaufsaugenden Material vorhanden, vorzugsweise aus einem Abstandsgewirke aus Polyester, Polyamid und/oder Baumwolle, um den Tragekomfort des Patienten im Bereich der Achseln zu erhöhen.

Sowohl die Bänder 2 selbst, als auch die Klettenabschnitte 5 sowie die Polster 6 sind bevorzugt rechteckig geformt.

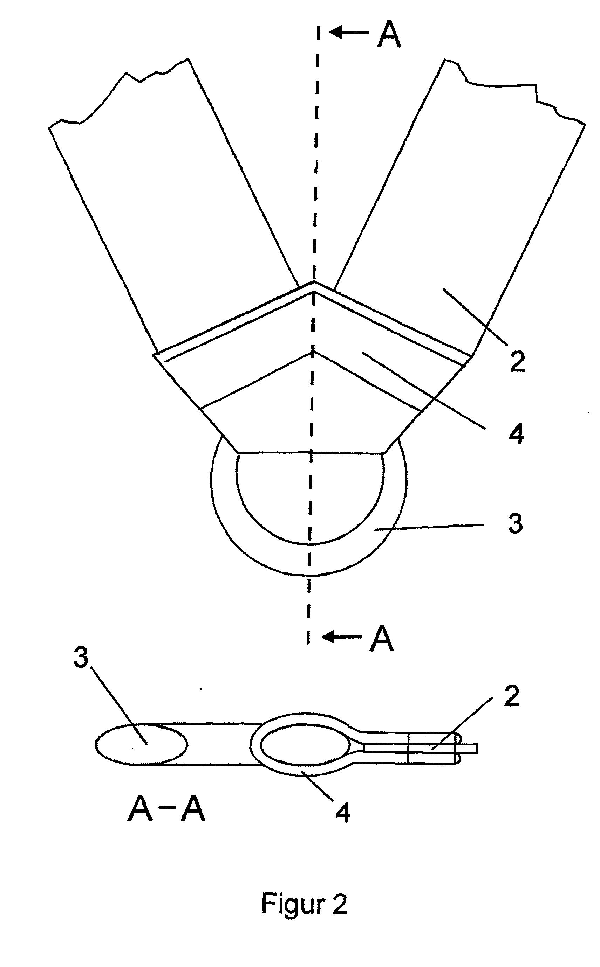

In der Figur 2 sind die pentagonal geformte Stofftasche 4, die die Bänder 2 am Ring 3 festlegt, im Detail dargestellt sowie ein seitlicher Schnitt gemäß der Linie A-A durch diesen Bereich der Bandage 1.

Die Stofftasche 4 besteht vorzugsweise aus einem einzigen Stoffstück, in das der Ring 3 gelegt wird, anschließend wird das Stoffstück zur Bildung der Stofftasche 4 umgeklappt. Vor dem Zunähen der Stofftasche 4 werden die beiden Bänder 2 eingeführt und durch eine oder mehrere Nähte fixiert Die Bänder 2 liegen somit fest, ebenso ist der Winkel α vorgegeben. Der Ring 3 kann trotzdem in der Stofftasche 4 rotieren.

Des weiteren zeigt Figur 2 einen seitlichen Schnitt gemäß der Linie A-A durch die Bandage 1. Der Schnitt offenbart den elliptischen Querschnitt des Ringes 3 sowie die Anordnung der Bänder 2 in der Tasche 4.

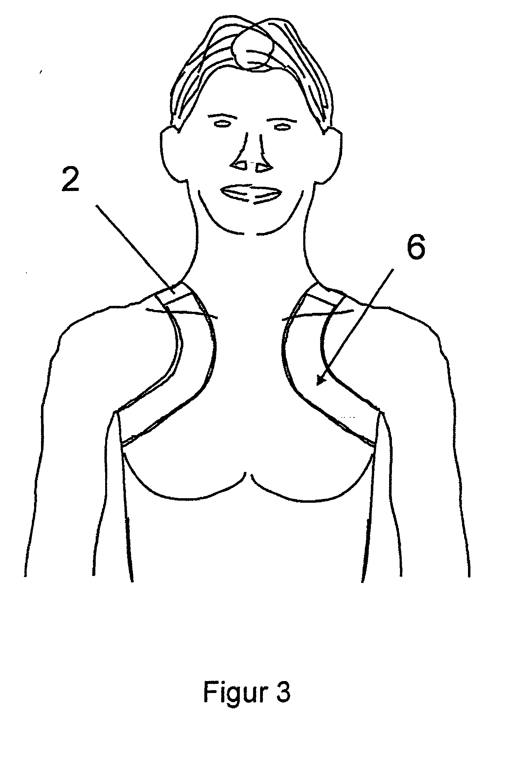

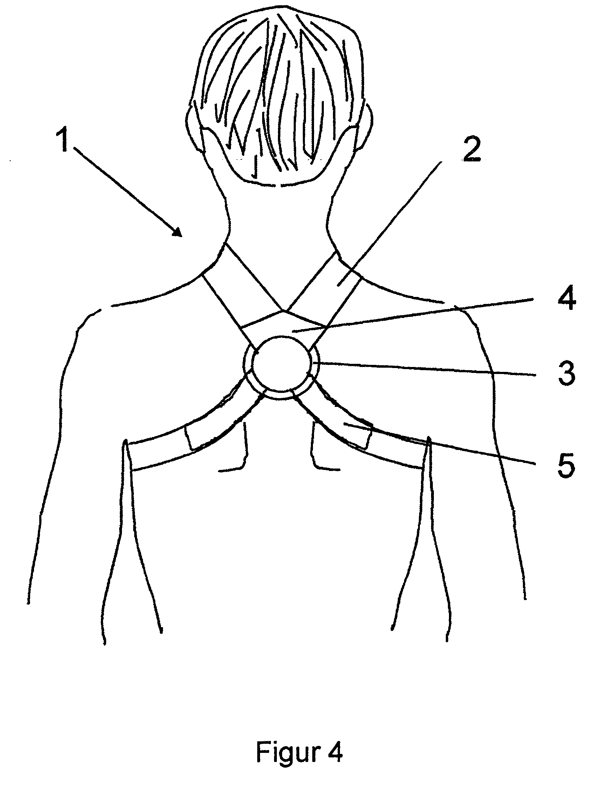

Die Figuren 3 und 4 zeigen schließlich die am menschlichen Körper angelegte Clavicula-Bandage 1 von anterior beziehungsweise posterior.
Das Anlegen der Bandage 1 erfolgt, indem der Ring 3 auf dem Rücken des Patienten plaziert wird und die freien Enden der Bänder 2 über die Schlüsselbeine gelegt werden. Die freien Enden der Bänder 2 werden unter den Achseln durch wieder zum Ring 3 geführt. Beginnend mit dem Band 2, das über dem unbeschadeten Schlüsselbein liegt, werden die Bänder 2 durch den Ring 3 gezogen, und zwar derartig, daß die Bänder 2 vom Körper weg bewegt werden. Die Enden der Bänder 2 werden schließlich mittels der Klettenden 5 auf den Bändern 2 selbst befestigt, so daß der Ring 3 durch die sich daraus ergebenden Schlaufen in seiner Lage auf dem Rücken festliegt.
Gegebenenfalls kann sich ein Nachjustieren der Bandage 1 ergeben, indem die Schlaufen nochmals kurz geöffnet werden, um den erforderlichen Druck und den erwünschten Sitz der Bandage zu gewährleisten.

## Patentansprüche

1. Clavicula-Bandage 1 mit zwei in ihrer Länge einstellbaren Bändern 2, die an ihren freien Enden Mittel 5 zur Schlaufenbildung aufweisen, wobei die Bänder 2, die im wesentlichen aus nicht dehnbarem Material bestehen, jeweils mit ihrem anderen Ende an einem Ring 3 befestigt sind **dadurch gekennzeichnet, daß** die Bänder 2 durch eine Stofftasche 4 in ihrer Lage zueinander fixiert sind und, daß der Ring 3 einen flachen elliptischen Querschnitt aufweist.

2. Bandage gemäß Anspruch 1, **dadurch gekennzeichnet, daß** die Mittel 5 zur Schlaufenbildung aus einem Klettverschlußsystem bestehen.

3. Bandage gemäß Anspruch 2, **dadurch gekennzeichnet, daß** das Klettverschlußsystem in der Weise ausgebildet ist, daß sich am freien Ende der Bänder 2 auf ihrer körperabgewandten Außenfläche ein Klettenabschnitt befindet, der das eine Verschlußteil des Klettverschlusses bildet und aus im Abstand voneinander angeordneten aufwärts gerichteten Häkchen besteht, und daß sich im Anschluß daran zumindest auf einem Teilabschnitt der Außenfläche klettbares Material befindet, welches als das andere Teil des Klettverschlusses fungiert, indem die Häkchen auf ihm zu haften vermögen.

4. Bandage gemäß den Ansprüchen 1 bis 3, **dadurch gekennzeichnet, daß** die Stofftasche 4 die Form eines nicht regelmäßigen Pentagons aufweist, so daß der Winkel α zwischen den sich vom Ring 3 entfernenden Bändem 2 50° bis 95° beträgt, insbesondere 68°.

5. Bandage gemäß den Ansprüchen 1 bis 4, **dadurch gekennzeichnet, daß** die Bänder 2 etwa im Mittelabschnitt ihrer körperzugewandten Innenseite eine Polsterung 6 aus einem schweißaufsaugenden Material insbesondere aus Polyester, Polyamid und/oder Baumwolle aufweisen.

6. Bandage gemäß Anspruch 5, **dadurch gekennzeichnet, daß** die Polsterung 6 aus einem Abstandsgewirke mit einer Stärke von 3,5 mm bis 15 mm und einem Flächengewicht von 200 bis 1000 g/m² besteht, wobei insbesondere die Oberfläche der der Achselpartie zugewandten Seite der Polsterung 6 aufgerauht ist.

7. Bandage gemäß Anspruch 5, **dadurch gekennzeichnet, daß** die Polsterung 6 aus einem Abstandsgewirke mit einer zusätzlichen Lage der Achselpartie zugewandten Seite abgedeckt werden kann.

## Claims

1. Clavicle bandage 1 with two straps 2 of adjustable length which at their free ends have means 5 for forming a loop, and which straps 2 are made principally of non-stretchable material and are each secured at their other end to a ring 3, **characterized in that** the straps 2 are fixed in their position relative to one another by a material pocket 4, and **in that** the ring 3 has a flat elliptical cross section.

2. Bandage according to Claim 1, **characterized in that** the means 5 for forming a loop consist of a touch-and-close system.

3. Bandage according to Claim 2, **characterized in that** the touch-and-close system is designed such that the free end of the straps 2 has, on its outer surface facing away from the body, a touch-and-close component forming one closure part of the touch-and-close system and consisting of upwardly directed hooks arranged at a distance from one another, and **in that** at least a subsection of the outer surface has a touch-and-close material which functions as the other part of the touch-and-close system onto which the hooks are able to adhere.

4. Bandage according to Claims 1 to 3, **characterized in that** the material pocket 4 has the shape of an irregular pentagon, so that the angle α between the straps 2 extending away from the ring 3 is 50° to 95°, in particular 68°.

5. Bandage according to Claims 1 to 4, **characterized in that** the straps 2, approximately in the middle section of their inner side facing towards the body, have a padding 6 of sweat-absorbing material, in particular polyester, polyamide and/or cotton.

6. Bandage according to Claim 5, **characterized in that** the padding 6 consists of a spacer knit with a thickness of from 3.5 mm to 15 mm and a weight per unit area of from 200 to 1000 g/m², and the surface of that side of the padding 6 facing towards the shoulder region is roughened.

7. Bandage according to Claim 5, **characterized in that** the padding 6 consisting of a spacer knit can be covered with an additional ply on the side facing the shoulder region.

## Revendications

1. Bandage claviculaire 1 avec deux bandes 2 ajustables en longueur qui présentent à leurs extrémités libres des moyens 5 pour former une boucle, les bandes 2 se composant essentiellement de matériau non extensible étant fixées à chaque fois par leur autre extrémité à un anneau 3, **caractérisé en ce que** les bandes 2 sont fixées en position l'une par rapport à l'autre par une poche en textile 4, et **en ce que** l'anneau 3 présente une section transversale plate elliptique.

2. Bandage selon la revendication 1, **caractérisé en ce que** les moyens 5 pour former une boucle se composent d'un système de fermeture velcro.

3. Bandage selon la revendication 2, **caractérisé en ce que** le système de fermeture velcro est réalisé de telle manière qu'une portion velcro se trouve à l'extrémité libre des bandes 2 sur leur face extérieure opposée au corps, laquelle portion forme une partie de la fermeture velcro et se compose de petits crochets orientés vers le haut et espacés les uns des autres, et **en ce qu'**à la suite de celle-ci, au moins sur une partie partielle de la surface extérieure, on prévoit du matériau accrochable qui sert d'autre partie de la fermeture velcro, les crochets pouvant adhérer sur celle-ci.

4. Bandage selon les revendications 1 à 3, **caractérisé en ce que** la poche en tissu 4 présente la forme d'un pentagone irrégulier de sorte que l'angle α entre les bandes 2 s'éloignant de l'anneau 3 soit compris entre 50° et 95°, en particulier soit de 68°.

5. Bandage selon les revendications 1 à 4, **caractérisé en ce que** les bandes 2 présentent, approximativement dans la portion centrale de son côté intérieure tourné vers le corps, un rembourrage 6 en un matériau absorbant la transpiration, en particulier en polyester, en polyamide et/ou en coton.

6. Bandage selon la revendication 5, **caractérisé en ce que** le rembourrage 6 se compose d'un tissu à mailles d'espacement ayant une épaisseur de 3,5 mm à 15 mm et un grammage de 200 à 1000 g/m² , la surface du côté du rembourrage 6 tourné vers la partie de l'aisselle étant notamment rendue rugueuse.

7. Bandage selon la revendication 5, **caractérisé en ce que** le rembourrage 6 en un tissu à mailles d'espacement peut être recouvert d'une couche supplémentaire du côté tourné vers la partie de l'aisselle.
